(19) Europäisches Patentamt
European Patent Office
Office européen des brevets

(11) Publication number : 0 393 529 B1

(12) EUROPEAN PATENT SPECIFICATION

(45) Date of publication of patent specification : 30.06.93 Bulletin 93/26

(21) Application number : 90107098.7

(22) Date of filing : 12.04.90

(51) Int. Cl.$^5$ : C07D 471/14, A61K 31/55, // (C07D471/14, 243:00, 221:00, 221:00)

# (54) 5,11-dihydro-6H-dipyrido [3,2-b:2',3'-e][1,4]diazepin-6-ones and their use in the prevention or treatment of AIDS.

(30) Priority : 20.04.89 US 340970

(43) Date of publication of application : 24.10.90 Bulletin 90/43

(45) Publication of the grant of the patent : 30.06.93 Bulletin 93/26

(84) Designated Contracting States : AT BE CH DE DK ES FR GB GR IT LI LU NL SE

(56) References cited :
DE-A- 1 936 670
FR-A- 1 542 160
CHEMICAL ABSTRACTS, vol. 90, no. 17, April 23, 1979, Columbus, Ohio, US; DR. KARL THOMAE GMBH: "11-Substituted 5,11-dihydro-6H-pyrido-(2,3-b)(1,4)benzodiazepin-6-ones." page 522, column 1, abstract-no. 137 875s
CHEMICAL ABSTRACTS, vol. 90, no. 19, May 7, 1979, Columbus, Ohio, US; BREWSTER, KEITH et al.: "The synthesis of some pyrido(1,4)benzoxazepines and a dipyrido(1,4) oxazepine." page 611, column 2, abstract-no. 152 145x
CHEMICAL ABSTRACTS, vol. 71, no. 23, December 8, 1969, Columbus, Ohio, US; SCHMIDT, GUENTHER et al.: "Analgesic 5,11-dihydro-6H-pyrido(2,3-b)(1,4)benzodiazepin-6-ones." page 394, column 1, abstract-no. 112 994z

(73) Proprietor : Boehringer Ingelheim Pharmaceuticals Inc.
90 East Ridge P.O. Box 368
Ridgefield Connecticut 06877 (US)
Proprietor : Dr. Karl Thomae GmbH
Postfach 1755
W-7950 Biberach 1 (DE)

(72) Inventor : Hargrave, Karl D., Dr.
4 Edna Drive
Brookfield, Connecticut 06805 (US)
Inventor : Schmidt, Günther, Dr.
(deceased on 08.08.1986)
-- (DE)
Inventor : Engel, Wolfhard, Dr.
Mozartstrasse 13
W-7950 Biberach 1 (DE)
Inventor : Trummlitz, Günter, Dr.
Buchenweg 27
W-7951 Warthausen (DE)
Inventor : Eberlein, Wolfgang, Dr.
Obere Au 6
W-7950 Biberach 1 (DE)

(74) Representative : Laudien, Dieter, Dr. et al
Boehringer Ingelheim GmbH, Abteilung Patente
W-6507 Ingelheim am Rhein (DE)

EP 0 393 529 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Field of the Invention

The invention relates to 5,11-dihydro-6H-dipyrido[3,2-b: 2',3'-e][1,4]diazepin-6-ones and pharmaceutically acceptable acid additions salts thereof, methods for preparing these compounds, the use of these compounds in the prevention or treatment of AIDS, and to pharmaceutical compositions containing these compounds.

### Background of the Invention

The human disease, Acquired Immune Deficiency Syndrome (AIDS), is caused by the Human Immunodeficiency Virus (HIV), particularly the strain known as HIV-1.

Like other viruses, HIV-1 cannot replicate without commandeering the biosynthetic apparatus of the host cell it infects. It causes this apparatus to produce the structural proteins which make up the viral progeny. These proteins are coded for by the genetic material contained within the infecting virus particle, or virion. Being a retrovirus, however, the genetic material of HIV is RNA, not DNA as in the host cell's genome. Accordingly, the viral RNA must first be converted into DNA, and then integrated into the host cell's genome, in order for the host cell to produce the required viral proteins.

The conversion of the RNA to DNA is accomplished through the use of the enzyme reverse transcriptase (RT), which is included within the infecting virion along with the RNA. Reverse transcriptase has three enzymatic functions; it acts as an RNA-dependent DNA polymerase, as a ribonuclease, and as a DNA-dependent DNA polymerase. Acting first as an RNA-dependent DNA polymerase, RT makes a single-stranded DNA copy of the viral RNA. Next, acting as a ribonuclease, RT frees the DNA just produced from the original viral RNA and then destroys the original RNA. Finally, again acting as a DNA-dependent DNA polymerase, RT makes a second, complementary DNA strand, using the first DNA strand as a template. The two strands form double-stranded DNA, which is integrated into the host cell's genome by another enzyme called an integrase.

Compounds which inhibit the enzymatic functions of HIV-1 reverse transcriptase will inhibit replication of HIV-1 in infected cells. Such compounds are useful in the prevention or treatment of HIV-1 infection in human subjects.

### Description of the Invention

In its composition of matter aspect, the invention comprises 5,11-dihydro-6H-dipyrido[3,2-b: 2',3'-e][1,4]diazepin-6-ones of the formula I

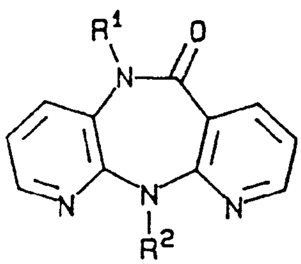

I

wherein,
$R^1$ and $R^2$ are the same or different and can be hydrogen or straight or branched alkyl of 1 to 5 carbon atoms, and pharmaceutically acceptable acid addition salts thereof.

The compounds of Formula I can be prepared by known methods or obvious modifications thereof. Methods A, B, C, and D, described below, are illustrative of the methods for preparing the compounds.

### Method A

Compounds of the general formula Ia

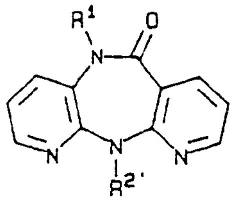

Ia

wherein $R^1$ has the meanings mentioned above and $R^{2'}$ has the meanings of $R^2$ with the exception of hydrogen, can be obtained by cyclizing carboxylic acid amides of general formula II

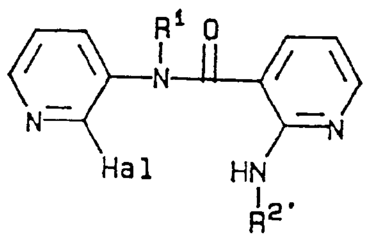

II

wherein $R^1$ and $R^{2'}$ have the same meanings set forth with respect to Formula Ia and Hal represents fluorine, chlorine, bromine or iodine. Cyclisation is preferably carried out by converting the compounds of general formula II into their alkaline metal salts and subsequent condensation at temperatures between 40 and 150°C preferably at 50 to 80°C.

If in the starting compounds of general formula II, $R^1$ is different from hydrogen, metallation requires at least 1 mole of the metallating agent. If on the other hand, $R^1$ is hydrogen, at least 2 moles of this agent must to be used. For metallation lithium, sodium and potassium hydrides, lithium alkyls, such as n-butyl lithium, are preferably used.

The reaction is usually carried out in inert solvents, e.g. in tetrahydrofuran, 1,4-dioxane, glycoldimethyl ether, diethyleneglycoldimethyl ether, triethyleneglycoldimethyl ether, dimethylformamide, benzene or anisole. Cyclisation may also be effected by heating carboxylic acid amides of general formula II in dipolar aprotic solvents, preferably in sulfolane or dimethylsulfone, whereby catalytic quantities of strong acides, e.g. sulfuric acid, hydrochloric acid, hydrobromic acid, phosphoric acid, polyphosphoric acid, methansulfonic acid or p-toluenesulfonic acid, proved to be of advantageous. The necessary reaction temperature is between 110 and 220°C, the preferred range of temperature being between 130 and 170°C.

Method B

Compounds of general formula Ib

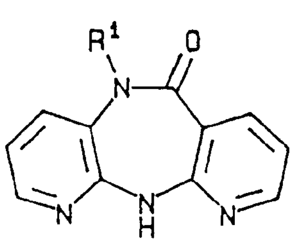

(Ib)

wherein $R^1$ is defined as above, can be prepared by hydrolytic cleavage of the arylmethyl group in compounds of general formula III

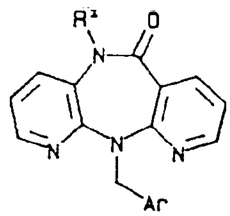

(III)

wherein $R^1$ is defined as mentioned above and Ar can be a phenyl or 4-methoxyphenyl group. Hydrolysis is effected by strong acids or Lewis-acids at temperatures between -20 and +150°C. Such acids can be sulfuric acid, methanesulfonic acid, trifluoroacetic acid, trifluoromethanesulfonic acid, phosphoric or polyphosphoric acid. When using phosphoric or polyphosphoric acid, the addition of solvents such as benzene, toluene, phenol, anisole or veratrole proved to be of advantage.

If Lewis acids, such as aluminium chloride or bromide are used to eliminate the arylmethyl group, solvents such as aromatic hydrocarbons, e.g. benzene, toluene, anisole, or mixtures thereof with dichloromethane are suitable.

Method C

Compounds of general formula Ic

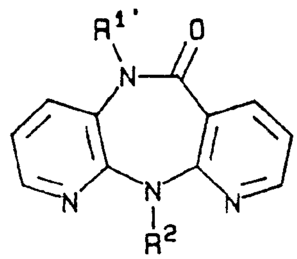

(Ic)

wherein $R^{1'}$, has the meanings of $R^1$ with the exception of hydrogen and $R^2$ is defined as above, may be obtained by converting a 5,11-dihydro-6H-dipyrido[3,2-b2',3'-e][1,4]diazepin-6-one of the formula IV

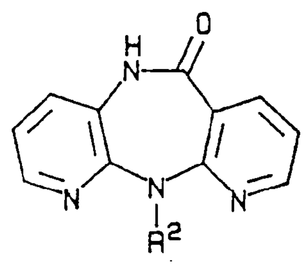

(IV)

wherein $R^2$ is defined as above, into the corresponding 5-alkali or alkaline earth metal compound and subsequently reacting the alkali metal compound with a reactive ester of the formula V

$R^{1'}X$ (V)

wherein $R^{1'}$ has the same meanings as in formula Ic and X is the radical of a reactive ester, a halogen atom, the group $OSO_2OR^{1'}$, the methanesulfonyloxy or ethanesulfonyloxy group or an aromatic sulfonyloxy group. Instead of converting the compound of the general formula IV to its corresponding alkali metal salt in the first step, the alkylation of a compound of formula IV may also be performed by reaction with a compound of formula V in the presence of amines, such as triethylamine, diazabicycloundecene or 4-(dimethylamino)pyridine, or of alkali carbonates or bicarbonates, such as sodium and potassium carbonate or sodium bicarbonate.

The conversion of a compound of general formula IV into the corresponding alkali metal or alkaline earth metal compound may be effected by reacting a compound of formula IV with an alkali metal or alkaline earth metal hydroxide, such as lithium hydroxide, barium hydroxide, sodium hydroxide or potassium hydroxide, with an alkali metal alcoholate, such as sodium methanolate or potassium tert-butoxide, with an alkali metal amide, such as sodium amide or potassium amide, or with an alkali metal hydride such as sodium hydride or potassium

hydride. The reaction is preferably carried out at elevated temperatures and in the presence of a suitable organic solvent. Inert organic solvents, such as tetrahydrofuran or glycoldimethyl ether are preferred if alkali metal hydrides are used as the metallating agents, whereas, if an alkali or alkaline earth metal hydroxide is used an aqueous mixture with an organic solvent, such as methanol or tetrahydrofuran, may also be employed. For conversion of the alkali or alkaline earth metal-substituted 5,11-dihydro-6H-dipyrido[3,2-b:2',3'-e][1,4]diazepin-6-one thus obtained into a compound of general formula Ic, the solution or suspension of the alkali or alkaline earth metal compound is reacted directly, i.e. without isolation, with a compound of formula V at room temperature or at elevated temperatures, preferably at the boiling point of the solvent or reaction medium, whichever is lower. The substitution takes place almost exclusively at the nitrogen atom in the 5-position of the dihydrodipyridodiazepinone, even if $R^2$ in the starting material of formula IV is a hydrogen atom, provided that one equivalent of base and one equivalent of a compound of formula V are used. The substitution product of general formula Ic may, if desired, be purified via conversion to an acid addition salt thereof, which may be formed by conventional methods.

Method D

Compounds of general formula Id

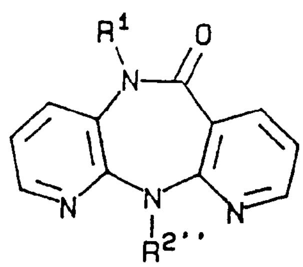

(Id)

wherein $R^{2''}$ represents a straight or branched alkyl group of 1 to 5 carbon atoms and $R^1$ represents the groups mentioned above, can be obtained by converting 5,11-dihydro-6H-dipyrido[3,2-b:2',3'-3][1,4]diazepin-6-ones of general formula Ib into the corresponding metal salts of general formula VIa or - in case of $R^1$ in the compound of formula Ib being hydrogen - of formula VIb

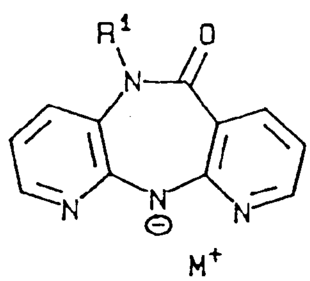

VIa

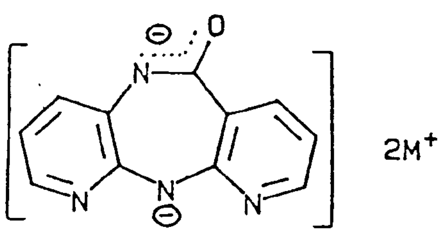

VIb

wherein $R^1$ is as hereinbefore defined and M represents an alkali metal, such as lithium, sodium, potassium, rubidium or caesium, or M represents the group MgHal, wherein Hal is a chlorine, bromine or iodine atom, and subsequently alkylating with a compound of general formula VII

$R^{2''}X$ (VII)

wherein $R^{2''}$ and X are as hereinbefore defined.

The conversion of a compound of general formula Ib into the corresponding alkali metal compound of formulae VIa and VIb may be effected by reacting a compound of formula Ib with a lithium alkyl (e.g. n-butyl lithium, or t-butyl lithium) optionally in the presence of tetramethylethylenediamine, a lithium dialkylamide, (e.g. lithium diisopropylamide, lithium dicyclohexylamide and lithium isopropyl-cyclohexylamide), a lithium aryl (e.g. phenyl lithium), alkali metal hydroxides (e.g. lithium, sodium or potassium hydroxide), alkali metal hydrides (e.g. sodium or potassium hydride) or alkali metal amides (e.g. sodium or potassium amides), or Grignard reagents (e.g. methyl magnesium iodide, ethyl magnesium bromide or phenyl magnesium bromide). One equivalent of base is required for the formation of compounds of formula VIa, whereas two equivalents of base are required for the formation of compounds of formula VIb. The metallation is conveniently carried out in an inert organic

solvent at temperatures of between -100°C and the boiling point of the reaction mixture in question. If lithium alkyls, lithium aryls, lithium dialkylamides or Grignard reagents are used for the metallation, the preferred solvents are ethers such as tetrahydrofuran, diethyl ether or dioxane, optionally in a mixture with aliphatic or aromatic hydrocarbons, such as hexane or benzene, and the operation is carried out at temperatures of between -20 and +80°C. When metallation is effected with alkali metal hydrides and alkali metal amides, in addition to the solvents mentioned hereinbefore it is also possible to use xylene, toluene, acetonitrile, dimethylformamide and dimethylsulfoxide, whilst if alkali metal hydroxides are used it is also possible to use alcohols such as ethanol, methanol and aliphatic ketones such as acetone, as well as mixtures of these solvents with water.

For conversion of the alkali metal substituted 5,11-dihydro-6H-dipyrido[3,2-b;2',3'-e][1,4]diazepin-6-one thus obtained into a compound of formula Ib, the solution or suspension of the alkali metal compound is reacted directly, i.e. without isolation of the reaction product with a compound of formula VII at elevated temperatures, preferably at the boiling point of the solvent or suspension medium or at the boiling point of the compound VII, whichever is lower.

The carboxylic acid amides of general formula II used as starting materials are obtained for example by amination of 2-chloro-nicotinic acid amides of general formula VIII

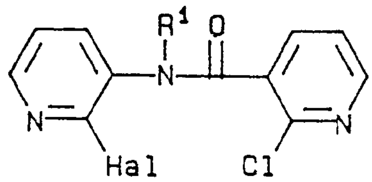

(VIII)

wherein $R^1$ and Hal are as hereinbefore defined, with primary amines of general formula IX

$$H_2N\text{-}R^{2'} \qquad (IX)$$

wherein $R^{2'}$ is as hereinbefore defined for this amination at least equivalent amounts of the amines of formula IX are used. The reaction can also be carried out in the presence of inorganic or organic auxiliary bases, such as triethylamine, N,N-dimethylaniline, sodium and potassium carbonate. The reaction can be carried out without using a solvent; it is of some advantage, however, to use inert organic solvents at temperatures of between 0°C and 150°C, preferably at reflux temperature. Suitable inert solvents that can be used include an excess of the primary amine of general formula IX, open chain or cyclic ethers, such as tetrahydrofuran, 1,4-dioxane, glycoldimethyl ether, diethyleneglycoldimethyl ether; aromatic hydrocarbons, such as benzene, toluene, xylene, chlorobenzene or pyridine; alcohols such as methanol, ethanol, isopropanol; dipolar aprotic solvents such as dimethylformamide; 1,3-dimethyl-2-imidazolidinone, 1,3-dimethyl-tetrahydro-2(1H)-pyrimidinone and sulfolane. Starting materials of general formula VIII, wherein $R^1$ is different from hydrogen, are prepared from 2-chloronicotinic acid amides of general formula X

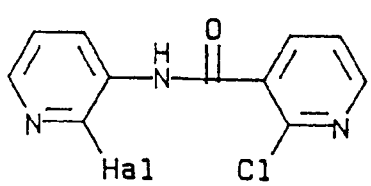

(X)

by reaction with alkylating agents of general formula V in the presence of proton acceptors, for example of amines, such as triethylamine, diazabicycloundecene, 4-(dimethylamino)pyridine, or alkali or alkaline earth metal hydroxides, such as sodium hydroxide, potassium hydroxide, calcium hydroxide, of alkali carbonates, or alkaline earth metal carbonates or hydrogencarbonates, such as sodium carbonate or potassium carbonate, or potassium hydrogen carbonate.

2-Chloronicotinic acid amides of general formula X can be obtained by condensation of 2-chloronicotinic acid chloride with 3-amino-2-halogen-pyridines under well known reaction conditions.

All the other starting materials are known from the literature or may be purchased or may be obtained by procedures known from the literature.

Compounds of the formula I may, if desired, be converted into their non-toxic, pharmaceutically acceptable acid addition salts by conventional methods; for example, by dissolving the free base compound I in a suitable solvent and acidifying the solution with one or more molar equivalents of the desired acid.

Examples of inorganic and organic acids which will form nontoxic pharmaceutically acceptable acid addition salts with a compound of the formula I are the following: hydrochloric acid, hydrobromic acid, sulfuric acid, phosphoric acid, nitric acid, tartaric acid, citric acid, methanesulfonic acid, 8-chloro-theophylline and the like. Compounds of the general formula I usually form acid addition salts with one molar equivalent of the acid.

The above described compounds of Formula I possess inhibitory activity against HIV-1 reverse transcriptase when administered in suitable dosage forms. They are useful in the prevention or treatment of AIDS, ARC and related disorders associated with HIV infection. Another aspect of the invention, therefore, is a method for preventing or treating HIV-1 infection which comprises administering to a human being, exposed to or infected by HIV-1, a prophylactic or therapeutic amount of a novel compound of Formula 1, as described above.

The compounds of formula I may be administered in single or divided doses by the oral, parenteral or topical routes. A suitable oral dosage for a compound of formula I would be in the range of about 10 to 500 mg per day. In parenteral formulations, a suitable dosage unit may contain from 1 to 50 mg of said compounds, whereas for topical administration, formulations containing 0.01 to 1% active ingredient are preferred. It should be understood, however, that the dosage administration from patient to patient will vary and the dosage for any particular patient will depend upon the clinician's judgement, who will use as criteria for fixing a proper dosage the size and condition of the patient as well as the patient's response to the drug.

When the compounds of the present invention are to be administerted by the oral route, they may be administered as medicaments in the form of pharmaceutical preparations which contain them in association with a compatible pharmaceutical carrier material. Such carrier material can be an inert organic or inorganic carrier material suitable for oral administration. Examples of such carrier materials are water, gelatin, talc, starch, magnesium stearate, gum arabic, vegetable oils, polyalkylene-glycols or petroleum jelly.

The pharmaceutical preparations can be prepared in a conventional manner and finished dosage forms can be solid dosage forms, for example, tablets, dragees or capsules, or liquid dosage forms, for example solutions, suspensions or emulsions. The pharmaceutical preparations may be subjected to conventional pharmaceutical operations such as sterilization. Further, the pharmaceutical preparations may contain conventional adjuvants such as preservatives, stabilizers, emulsifiers, flavor-improvers, wetting agents, buffers or salts for varying the osmotic pressure. Solid carrier material which can be used include, for example, starch, lactose, mannitol, methyl cellulose, microcrystalline cellulose, talc, silica, dibasic calcium phosphate, and high molecular weight polymers (such as polyethylene glycol).

For parenteral use, it is preferred to administer a compound of formula I in an aqueous or non-aqueous solution, suspension or emulsion in a pharmaceutically acceptable oil or a mixture of liquids, which may contain bacteriostatic agents, antioxidants, preservatives, buffers or other solutes to render the solution isotonic with the blood, thickening agents, suspending agents or other pharmaceutically acceptable additives. Additives of this type include, for example, tartrate, citrate and acetate buffers, ethanol, propylene glycol, polyethylene glycol, complex formers (such as EDTA), antioxidants (such as sodium bisulfite, sodium metabisulfite, and ascorbic acid), high molecular weight polymers (such as liquid polyethylene oxides) for viscosity regulation and polyethylene derivatives of sorbitol anhydrides. Preservatives may also be added if necessary, such as benzoic acid, methyl or propyl paraben, benzalkonium chloride and other quaternary ammonium compounds.

The compounds of this invention may also be administered as solutions for nasal application and may contain in addition to the compounds of this invention suitable buffers, tonicity adjusters, microbial preservatives antioxidants and viscosity-increasing agents in an aqueous vehicle. Examples of agents used to increase viscosity are polyvinyl alcohol, cellulose derivatives, polyvinylpyrrolidone, polysorbates or glycerin. Microbial preservatives added may include benzalkonium chloride, thimerosal, chlorobutanol or phenylethyl alcohol.

Additionally, the compounds provided by the invention can also be administered by suppository.

As stated before, the compounds provided by the invention inhibit the enzymatic activity of HIV-1 RT. Based upon fairly extensive testing of these compounds, it is known that the RNA-dependent DNA polymerase activity of HIV RT is inhibited. Based upon less extensive testing, it is believed that the DNA-dependent DNA polymerase activity is also inhibited.

Utilizing the Reverse Transcriptase (RT) Assay described below, compounds can be tested for their ability to inhibit the RNA-dependent DNA polymerase activity of HIV RT. Certain specific compounds described in the Examples which appear below, were so tested. The results of this testing appears in Table I, below.

REVERSE TRANSCRIPTASE (RT) ASSAY

Assay Theory:

Among the enzymes for which Human Innunodeficiency Virus (HIV-1) encodes is a reverse transcriptase (1), so-named because it transcribes a DNA copy from an RNA template. This activity can be quantitatively measured in a cell-free enzyme assay which has been previously described (2), and is based upon the observation that reverse transcriptase is able to use a synthetic template [poly r(C) primed with oligo d(G)] to transcribe a radio-labelled, acid-precipitable DNA strand utilizing $^3$H-dGTP as a substrate.

Materials:

a) Preparation of the enzyme

Reverse transcriptase enzyme from the LAV strain of Human Immunodeficiency Virus (HIV-1) (1) was isolated from the bacterial strain JM109 (3) expressing the DNA clone pBRTprt1+ (2) which is under the control of the lac promotor in the expression vector pIBI21 (4). An overnight culture grown in 2XYT medium (37oC, 225 rpm) (5) supplemented with 100 µg/ml ampicillin for positive selection is inoculated at a 1:40 dilution into M9 medium supplemented with 10µg/ml thiamine, 0.5% casamino acids, and 50 µg/ml ampicillin (5). The culture is incubated (37°/c, 225 rpm) until it reaches an OD540 of 0.3-0.4. At that time the repressor inhibitor IPTG (isopropyl b-D-thiogalactopyranoside) is added to 0.5mM and incubated for 2 additional hours. Bacteria are pelletted, resuspended in a 50mM Tris, 0.6mM EDTA, 0.375M NaCl buffer and digested by the addition of lysozyme (1mg/ml) for 30 minutes on ice. The cells are lysed by the addition to 0.2% NP-40 and brought to 1M NaCl.

After removal of the insoluble debris by centrifugation, the protein is precipitated by the addition of 3 volumes of saturated aqueous ammonium sulfate. The enzyme is pelleted, resuspended in RT (50mM Tris pH 7.5, 1mM EDTA, 5mM DTT, 0.1% NP-40, 0.1M NaCl, and 50% glycerol) buffer, and stored at 70° for further use.

b) Composition of 2X concentrated stock reaction mixture

| Stock Reagent | 2X Mix Concentrtation |
|---|---|
| 1M Tris pH 7.4 | 100mM |
| 1M Dithiothrietol | 40mM |
| 1M NaCl | 120mM |
| 1% Nonidet P-40 | 0.1% |
| 1M MgCl | 4mM |
| [poly r(C) /oligo d(G)](5:1) | 2µg/ml |
| $^3$H-dGTP (81µM) | 0.6µM |

Assay Procedure:

The 2X concentrated stock reaction mixture is aliquoted and stored at -20°C. The mixture is stable and thawed for use in each assay. This enzyme assay has been adapted to a 96 well microtiter plate system, and has been previously described (6). Tris buffer (50 mM, pH 7.4), vehicle (solvent diluted to match the compound dilution), or compounds in vehicle are dispensed into 96-well microtiter plates (10µl/well; 3 wells/compound). The HIV RT enzyme is thawed and diluted in 50mM Tris pH 7.4 so that fifteen ul of diluted enzyme contain 0.001 Unit (one unit is that amount of enzyme to transform 1 micromole of substrate per minute at 25°C) and are dispensed per well. Twenty µl of 0.12-0.5M EDTA are added to the first three wells of the microtiter plate.

EDTA chelates the $Mg^{++}$ present and prevents reverse transcription. This group serves as background polymerization which is subtracted from all other groups. Twenty-five µl of the 2X reaction mixture are added to

all wells and the assay is allowed to incubate at room temperature for 60 minutes. The assay is terminated by precipitating the DNA in each well with 50µl of 10% trichloracetic acid (TCA) in 1% sodium pyrophosphate. The microtiter plate is incubated for 15 minutes at 4°C and the precipitate is fixed onto #30 glass fiber paper (Schleicher & Schuell) using a Skatron semi-automatic harvester. The filters are then washed with additional 5% TCA containing 1% sodium pyrophosphate, rinsed with 70% aqueous ethanol, dried, and transferred to scintillation vials (6). Each vial receives 2 mls of scintillation cocktail and is counted in a Beckman beta counter.

Calculations for percent inhibition are as follows:

$$\%\text{inhibition} = \frac{\text{CPM Mean Test Value} - \text{CPM Mean Control Value X100}}{\text{CPM Mean Control Value}}$$

References:

1. Benn, S., et al., SCIENCE 230:949, 1985
2. Farmerie, W.G. et. al., SCIENCE 236: 305, 1987
3. Yanisch-Perron, C., Viera, J., and Messing, J., GENE 33:103,1985
4. International Biotechnologies, Inc., P.O. Box 9558, New Haven, CT 06535
5. Maniatis, T, Fritsch, E.F., and J. Sambrook, eds. MOLECULAR CLONING: A LABORATORY MANUAL, Cold Spring Harbor Laboratory, 1982
6. Spira, T., et. al. J. Clinical Microbiology, 25:97, 1987.

In order to confirm that compounds which are active in the RT Assay also have the ability to inhibit HIV replication in a living system, compounds according to the invention were also tested in the human T-Cell Culture Assay described below. The results of this testing appear in Table I.

HUMAN T CELL CULTURE ASSAY

Assay Theory: Formation of syncytia is a feature of in vitro cultures of CD4+ T-cells infected with HIV-1. In this assay, T-cells are treated with a putative replication inhibiting compound and then infected with HIV-1. After incubation the culture is checked for the formation of syncytia. The absence or reduction in the number of syncytia is used as a measure of the test compound's ability to inhibit HIV replication.

Assay Method: The target cells, designated C8166, are a subclone of human lymphoma cells of T-cell origin and are established at an initial density of $5x10^4$ per 100 ul in RPMI 1640 (+ 10% fetal bovine serum) culture medium in 96 well flat bottom plates. A selected amount of test compound, dissolved in DMSO is included. After 24 hours, 50-100 $TCID_{50's}$ (the dose that results in induced effect in 50% of test cultures) of the HTLV-IIIB strain of HIV-1 (2) are innoculated into each culture. Control cultures receive compound or virus only. Four days after virus challenge, cultures are visually examined for the frequency and distribution of virus-induced giant cell syncytia. The percent inhibition by the test compound is determined by comparison with control values. Confirmation of the presence or absence of virus replication is accomplished by harvesting the cell free culture fluids from all experimental groups to determine the presence or absence of infectious progeny through the induction of syncytia formation in secondary human T-cell cultures after 3 days.

REFERENCES:

(1) M. Somasundaran and H.L. Robinson, Science 242, 1554 (1998)
(2) G.M. Shaw, A.H. Hahn, S.K Arya, J.E. Groopman, R.C. Gallo and F. Wong-Staal, Science 226, 1165 (1984)

In order to assess the specificity of the enzyme inhibitory activity of the compounds provided by the invention, a few were tested, using known per se assay methods, for their ability to inhibit Feline Leukemia Virus-derived reverse transcriptase and Calf Thymus-derived DNA alpha-polymerase. None of the compounds so tested was observed to possess any inhibitory activity against these enzymes. These results indicate that the enzyme inhibitory activity of the compounds provided by the invention is directed rather specifically against HIV RT.

In order to roughly assess the cytotoxicity of the compounds provided by the invention, several such compounds were tested in the MTT Cellular Cytotoxicity Assay described below. The results of this testing are reported in Table I, below. Compounds having a relatively high $EC_{50}$ are preferred.

MTT ASSAY FOR CELLULAR CYTOTOXICITY

Assay Theory:

The MTT [3-(4,5-dimethylthiazol-2yl)-2,5 diphenyl tetrazolium bromide) assay is based on cleavage of tetrazolium bromide by metabolically active cells, resulting in a highly quantitative blue color. This assay has been previously described (1) but has been optimized for the purposes or the testing reported herein.

Assay Method:

The H9 cell line (2), an established human lymphoma suspension cell line grown in RPMI 1640 supplemented with 10% fetal bovine serum is used as the target cell line in the assay. Cells (100µl) are plated in microtest plate wells at a concentration of $10^5$ cells per ml in the presence of varying concentrations of inhibitor. The cells are incubated at 37°C in a humidified $CO_2$ incubator. Five days later, 20µl of MTT (5mg/ml in RPMI 1640, sonicated, 0.2 micron filtered, and stored at 4°C) is added to each well. After 4 hours additional incubation at 37°C, 60µl of Triton-X is added to each well and thoroughly mixed to aid the solubilization of the crystals. Absolute ethanol (5µl) is added to each well and the resulting mixture is incubated for 30 minutes at 60°C and immediately read on a plate reader (Dynatech) at a wavelength of 570nm.

Data from this assay are used to generate a nonlinear regression analysis which yields an $EC_{50}$, the highest non-toxic concentration.

References:

1. Mosmann, Tim, J. Immunol. Methods, 65:55, 1983.
2. Jacobs. J.P., J. Natl. Cancer Inst., 34:231, 1965.

TABLE I

| Compound of Example No. | RT Inhibition % @ 10µg/ml | T-Cell Culture Assay($IC_{50}$) | Cytotoxity Assay ($EC_{50}$) |
|---|---|---|---|
| 2 | 94 | NT | NT |
| 3 | 94 | NT | NT |
| 4 | 98 | 0.19mM | 92mM |
| 8 | 100 | NT | NT |

Note: NT - not tested

The following examples further illustrate the present invention and will enable others skilled in the art to understand it more completely.

Example 1

5,11-Dihydro-6H-dipyrido[3,2-b:2',3'-4][1,4]diazepin-6-one

a) 2-Chloro-N-(2-chloro-3-pyridinyl)-3-pyridinecarboxamide

In a three-necked round-bottomed flask, fitted with an efficient reflux condenser, mechanical stirrer and dropping funnel, were placed 215 g (1.672 mol) of 3-amino-2-chloropyridine, dissolved in a mixture of 400 ml dioxane, 500 ml cyclohexane and 130 ml pyridine. The solution of 299.2 g (1.7 mol) of freshly prepared 2-chloro-3-pyridinecarboxylic acid chloride in 200 ml dioxane was added at such a rate as to keep the vigorous reaction under control. Thereafter, the reaction mixture was allowed to cool to room temperature and the resulting crystalline precipitate was filtered off and washed successively with cyclohexane and ether.

The dark brown product was dissolved in 5 l of a 3% aqueous solution of sodium hydroxide. The resulting solution was treated with charcoal, suction filtered, and the filtrate was acidified by addition of 50% aqueous acetic acid. The resulting precipitate was collected by filtration and thoroughly washed with water. After being dried overnight in a stream of nitrogen at room temperature the almost colorless product had a m.p. of 156-159°C and was sufficiently pure for further reactions. The yield was 376.0 g (84% of theory).

b) N-(2-Chloro-3-pyridinyl)-2-[[(4-methoxyphenyl)methyl]amino]-3-pyridinecarboxamide

13.4 g (0.05 mol) of the product obtained in step a) were dissolved in 20 ml of xylene, and the resulting solution was admixed with 13.8 g (0.1 mol) of p-methoxybenzylamine. Thereafter, the mixture was refluxed for two hours. The reaction mixture was then evaporated invacuo, and the residue was purified by column chromatography on silica gel (0.2-0.5 mm) using dichloromethane/ethyl acetate 10/1 (v/v) as an eluent. Colorless crystals, melting at 122-124°C (after recrystallization from acetonitrile). The yield was 17.2 g (93% of theory).

c) 5,11-Dihydro-11-[(4-methoxyphenyl)methyl]-6H-dipyrido-[3,2-b:2',3'-e][1,4]diazepin-6-one

16.7 g (0.0453 mol) of the product obtained in step b) were dissolved in 150 ml of absolute dioxane, and the resulting solution was admixed with 6.7 g (0.14 mol) of a 50% dispersion of sodium hydride in mineral oil.

Thereafter, the mixture - while protected against the external atmosphere by a low flow of nitrogen - was refluxed until no starting material could be detected by TLC. The surplus of sodium hydride was decomposed by cautious addition of 10 ml of a mixture of methanol and tetrahydrofuran (50/50 v/v). The reaction mixture was neutralized by addition of acetic acid and then was evaporated in vacuo. The residue was purified by column chromatography on silica gel (0.2-0.5 mm) using successively dichloromethane-/ethyl acetate 10/1 (v/v) and dichloromethane/ethyl acetate 1/1 (v/v) as eluents. The crystalline product obtained by evaportion of suitable fractions was recrystallized from acetonitrile and 2-propanol. The product had a m.p. of 213-215°C and was identified as 5,11-dihydro-11-[(4-methoxyphenyl)methyl]-6H-dipyrido[3,2-b:2',3'-e][1,4]diazepin-6-one. The yield was 10.3 g (68% of theory). $R_F$ 0.7 (Macherey-Nagel, Polygram$^R$ SIL G/$UV_{254}$, precoated plastic sheets for TLC; dichloromethane/ethyl acetate 1/1 v/v).

d) 5,11-Dihydro-6H-dipyrido[3,2-b:2',3'-e][1,4]diazepin-6-one

10.0 g (0.3 mol) of the product obtained in step c) were dissolved in 50 ml of trifluoroacetic acid whereby the mixture became slightly warm. Thereafter, the reaction mixture was stirred at 60°C for 1 hour. No starting material could be detected by TLC at that time. The mixture was then evaporated in vacuo. The residue thus obtained was thoroughly stirred with 0.5% aqueous ammonia and then was filtered by suction. The raw product was recrystallized from 150 ml of dimethyl sulfoxide to provide colorless crystals of m.p. of > 340°C. The yield was 4.8 g (75% of theory).

$C_{11}H_8N_4O$ (212,21)

| | | | | | | |
|---|---|---|---|---|---|---|
| Calc.: | C | 62/26 | H | 3/80 | N | 26/40 |
| Found: | | 62/40 | | 3/98 | | 26/52 |

The product was identified as 5,11-dihydro-6H-dipyrido[3,2-b:2',3'-e][1,4]diazepin-6-one by IR and $^1H$-NMR spectra and MS.

Example 2

5,11-Dihydro-11-propyl-6H-dipyrido[3,2-b:2',3'-e][1,4]diazepin-6-one

a) N-(2-Chloro-3-pyridinyl)-2-(propylamino)-3-pyridinecarboxamine

26.8 g (0.1 mol) of 2-chloro-N-(2-chloro-3-pyridinyl)-3-pyridinecarboxamine were dissolved in 200 ml of dioxane, and the resulting solution was admixed with 21.4 g (0.362 mol) of propylamine. Thereafter, the mixture was shaken in a stainless steel pressure vessel at 150°C for 6 hours. The reaction mixture was then evaporated in vacuo, and the residue was purified by column chromatography on silica gel (0.2-0.5 mm) using successively dichlormethane/ethyl acetate 10/1 (v/v) and dichloromethane/cyclohexane-/ethyl acetate 1/2/1 (v/v/v) as eluents. The product obtained by evaporation was a highly viscous resin of

$R_F$ 0.3 (Macherey-Nagel, Polygram$^R$ SIL G/UV$_{254}$, pre-coated plastic sheets for TLC; dichloromethane/cyclohexane/ethyl acetate 1/2/1 (v/v/v)), that had a satisfactory quality for the following reaction.

b) 5,11-Dihydro-11-propyl-6H-dipyrido[3,2-b:2',3'-e][1,4]diazepin-6-one

Using a procedure analogous to that described in Example 1c), 5,11-dihydro-11-propyl-6H-dipyrido[3,2-b:2',3'-e][1,4]diazepin-6-one, m.p. 184-186°C (recrystallized from acetonitrile), was prepared from the product obtained in the above step a) and sodium hydride. The yield was 74% of theory.

Example 3

5,11-Dihydro-5-methyl-11-propyl-6H-dipyrido[3,2-b:2',3'-e][1,4]diazepin-6-one

a) 2-Chloro-N(-2-chloro-3-pyridinyl)-N-methyl-3-pyridine-carboxamide

A four-necked round bottomed flask, equipped with a mechanical stirrer, a dropping funnel, a thermometer and an efficient reflux condenser, was charged with 268.1 g (1.0 mol) of 2-chloro-N-(2-chloro-3-pyridinyl)-3-pyridinecarboxamide, 260 ml of 50% aqueous sodium hydroxide, 1500 ml of toluene and 8.0 g (0.0352 mol) of benzyltriethylammonium chloride. Stirring was begun and the solution of 134 ml (178.5 g, 1.415 mol) of dimethyl sulphate in 1 l of toluene was added dropwise over a period of about 3 hours, whereby the temperature rose to 50-60°C. After the addition of dimethyl sulfate was complete, stirring at 60°C was continued for further 2 hours.

The reaction mixture was cooled to room temperature and 1 l of water was added. The layers were separated, and the aqueous phase was extracted three times with 300 ml portions of toluene. The organic layers were combined and washed successively with 300 ml of water, 300 ml of 1% aqueous acetic acid and 300 ml of water. The combined organic extracts were dried over sodium sulfate, and the solvent was removed by distillation under reduced pressure. The residue was purified by column chromatography on silica gel (0.2-0.5 mm) using as eluents successively toluene and ethyl acetate/cyclohexane/tetrahydrofuran 1/9/10 (v/v/v). The product obtained by evaporation of suitable fractions was recrystallized from acetonitrile/tert-butyl methyl ether 1/1 (v/v). It was highly soluble in dichloromethane, had a m.p. of 98-101°C and was identified to be 2-chloro-N-(2-chloro-3-pyridinyl)-N-methyl-3-pyridinecarboxamide. The yield was 232.5 g (82.5% of theory).

b) 5,11-Dihydro-5-methyl-11-propyl-6H-dipyrido[3,2-b:2',3'-e][1,4]diazepin-6-one

Using a procedure analogous to that described in Example 1c) except employing tetrahydrofuran instead of dioxane as a solvent and applying only equimolar quantities of sodium hydride, 5,11-dihydro-5-methyl-11-propyl-6H-dipyrido[3,2b:2',3'-e][1,4]-diazepin-6-one, a highly viscous oil of $R_F$ 0.2 (Macherey-Nagel, Polygram$^R$ SIL G/UV$_{254}$; pre-coated plastic sheets for TLC; dichloromethane/ethyl acetate 9/1 v/v); 0.3 (dichloromethane/cyclohexane/ethyl acetate 1/2/1 v/v/v); 0.75 (dichloromethane/ethylacetate/cyclohexane/methanol/aqueous ammonia 3/5/1.5/0/46/0/46/0/06 v/v/v/v/v) was prepared from the product obtained in the above step a). The yield was 75% of theory.

Example 4

5,11-Diethyl-5,11-dihydro-6H-dipyrido[3,2-b:2',3'-e][1,4]diazepin-6-one

6.4 g (0.03 mol) of 5,11-dihydro-6H-dipyrido[3,2-b:2',3'-e][1,4]diazepin-6-one were dissolved in 100 ml of absolute dimethylformamide, and the resulting solution was admixed with 3.4 g (0.071 mol) of a 50% dispersion of sodium hydride in mineral oil. While protected against the external atmosphere by a flow of nitrogen the mixture was stirred at 50-70°C for 1 hour. After the evolution of hydrogen had ceased the mixture was cooled to 30°C and 10.9 g (0.07 mol) of ethyl iodide were added dropwise within 15 minutes. For completion of the exothermic reaction the mixture was heated at 80-90°C for a further hour. The solvent was removed by distillation under reduced pressure. The residue was admixed with water and the suspension thus obtained was exhaustively extracted with dichloromethane. The product obtained after usual work-up was recrystallized from 150 ml of isooctane. The product had a m.p. of 102-103°C and was identified as 5,11-diethyl-5,11-dihydro-6H-dipyrido[3,2-b:2',3'-e][1,4]-diazepin-6-one. The yield was 5.7 g (71% of theory).

Example 5

5,11-Dihydro-5-ethyl-6H-dipyrido[3,2-b:2',3'-e][1,4]diazepin-6-one

a) N-(2-Chloro-3-pyridinyl-2-[(phenylmethyl)amino]-3-pyridine carboxamide

Using a procedure analogous to that described in Example 1b) except employing diethyleneglycoldimethyl ether as a solvent instead of xylene, N-(2-chloro-3-pyridinyl)-2-[(phenylmethyl)amino]-3-pyridine carboxamide, m.p. 95-97°C (recrystallized from diethyleneglycoldimethyl ether), was prepared from 2-chloro-N-(2-chloro-3-pyridinyl)-3-pyridinecarboxamide and benzylamine. The yield was 72% of theory.

b) 5,11-Dihydro-11-(phenylmethyl)-6H-dipyrido[3,2-b:2',3'-e][1,4]diazepin-6-one

Using a procedure analogous to that described in Example 1c) except employing diethyleneglycoldimethyl ether as a solvent instead of dioxane, 5,11-dihydro-11-(phenylmethyl)-6H-dipyrido[3,2-b:2',3'-e][1,4]diazepin-6-one, m.p. 212-213°C (recrystallized from 1-propanol), $R_F$ 0.7 (Macherey-Negal, Polygram[R] SIL G/$UV_{254}$, pre-coated plastic sheets for TLC; dichloromethane/ethyl acetate 1/1 v/v) was prepared from the product obtained in step a) and sodium hydride. The yield was 61% of theory.

5,11-Dihydro-5-ethyl-11-(phenylmethyl)-6H-dipyrido[3,2-b:2',3'-e][1,4]diazepin-6-one

Using a procedure analogous to that described in Example 3a), 5,11-dihydro-5-ethyl-11-(phenylmethyl)-6H-dipyrido-3,2-b:2',3'-e][1,4]diazepin-6-one, m.p. 209-211°C (recrystallized from toluene/acetonitrile 1/1 v/v), $R_F$ 0.5 (Macherey-Nagel, Polygram[R] SIL G/$UV_{254}$, pre-coated plastic sheets for TLC; dichloromethane/methanol 99/1 v/v) was prepared from the product obtained in step b) and diethyl sulfate. The yield was 82% of theory.

d) 5,11-Dihydro-5-ethyl-6H-dipyrido[3,2-b:2',3'-e][1,4]diazepin-6-one

Using a procedure analogous to that described in Example 1d), except employing a pressure vessel instead of an open one and heating the mixture at 120°C for 10 hours, 5,11-dihydro-5-ethyl-6H-dipyrido[3,2-b:2',3'-e][1,4] diazepin-6-one, m.p. 161-163°C (recrystallized from isooctane/ethyl acetate 1/1 v/v), $R_F$ 0.3 (Macherey-Nagel, Polygram[R] SIL G/$UV_{254}$, pre-coated plastic sheets for TLC; dichloromethane/methanol 99/1 v/v) was prepared from the product obtained in step (c). The yield was 57% of theory.

Example 6

5,11-Dihydro-5-methyl-6H-dipyrido[3,2-b:2',3'-e][1,4]diazepin-6-one

a) N-(2-Chloro-3-pyridinyl)-N-methyl-2-[(phenylmethyl)amino]-3-pyridinecarboxamide

Using a procedure analogous to that described in Example 1b), N-(2-chloro-3-pyridinyl)-N-methyl-2-[(phenylmethyl)amino]-3-pyridinecarboxamide, m.p. 114-116°C (recrystallized from tert-butyl methyl ether), $R_F$ 0.75 (Macherey-Nagel,Polygram[R] SIL G/$UV_{254}$, pre-coated plastic sheets for TLC; dichloromethane/ethyl acetate 3/1 v/v) was prepared from 2-chloro-N-(2-chloro-3-pyridinyl)-N-methyl-3-pyridinecarboxamide and benzylamine. The yield was 87% of theory.

b) 5,11-Dihydro-5-methyl-11-(phenylmethyl)-6H-dipyrido[3,2-b:2',3'-e][1,4]diazepin-6-one

Using a procedure of analogous to that described in Example 3b), 5,11-dihydro-5-methyl-11-(phenylmethyl)-6H-dipyrido[3,2-b:2',3'-e][1,4]diazepin-6-one, m.p. 198-199°C (recrystallized from acetonitrile), $R_F$ 0.45 (Macherey-Nagel, Polygram[R] SIL G/$UV_{254}$, pre-coated plastic sheets for TLC; dichloromethane/cyclohexane/ethyl acetate 1/2/1 v/v/v) was prepared from the product obtained in step (a). The yield was 80% of theory.

c) 5,11-Dihydro-5-methyl-6H-dipyrido[3,2-b:2',3'-e][1,4]diazepin-6-one

A mixture consisting of 75.5 g (0.239 mol) of the product obtained in step b), 2.5 kg of polyphosphoric acid, and 425 ml of anisole was stirred at 140-160°C for 2 hours. While still hot, the reaction mixture was stirred into crushed ice. Thereafter, the mixture was made slightly alkaline by addition of aqueous ammonia and was then exhaustively extracted with dichloromethane. The combined organic layers were dried over sodium sulfate and evaporated in vacuo. The residue was chromatographed on silica gel (0.2-0.5 mm) using dichloromethane/ethyl acetate 1/1 (v/v) as an eluent. The product obtained by evaporation of suitable fractions was recrystallized from acetonitrile yielding 21.6 g (40% of theory) of colorless crystals having a m.p. of 236-237°C, $R_F$ 0,35 (Macherey-Nagel, Polygram[R] SIL G/$UV_{254}$, pre-coated sheets for TLC; dichloromethane/ethyl acetate 1/1 v/v).

Example 7

5,11-Dihydro-6H-dipyrido[3,2-b:2',3'-e][1,4]diazepin-6-one

3.8 g (0.0126 mol) of the product obtained in Example 5b were dissolved in 20 ml of trifluoroacetic acid

whereby the mixture became slightly warm. Thereafter, the reaction mixture was refluxed for 8 hours. No starting material could be detected by TLC at that point of time. The mixture was then evaporated in vacuo, the residue thus obtained was thoroughly stirred with 0.5% aqueous ammonia and then filtered by suction. The raw material was suspended in 20 ml of acetonitrile, refluxed for 15 minutes and suction filtered while hot. The filter cake was recrystallized from hot dimethyl sulfoxide yielding 1.2 g (45% of theory) of colorless crystals which had a m.p. > 340°C and were identified by m.p., mixed m.p. and UV-, IR- and MS spectra to be identical with the compound obtained in Example 1d).

Example 8

5,11-Dihydro-11-ethyl-5-methyl-6H-dipyrido[3,2-b:2',3'-e][1,4]diazepin-6-one

a) 2-Chloro-N-(2-chloro-3-pyridinyl)-3-pyridinecarboxamide

Using a procedure analogous to that described in Example 1a), 2-chloro-N-(2-chloro-3-pyridinyl)-3-pyridinecarboxamide was prepared. The purified product was obtained by cooling the reaction mixture to room temperature and decanting the supernatant from the precipitate. The solid was then dissolved in methylene chloride, and the solution washed with water, dried (anhydrous sodium sulfate), and the solvent removed in vacuo. The solid was then washed with ethyl acetate and dried to provide 7.24g (84% of theory) of product suitable for use in the next reaction.

b) N-(2-Chloro-3-pyridinyl)-2-[[(4-methoxyphenyl)methyl]amino]-3-pyridinecarboxamide

Using a procedure analogous to that described in Example 1b), N-(2-chloro-3-pyridinyl)-2-[[(4-methoxyphenyl)methyl]amino]-3-pyridinecarboxamide was prepared. The purified product was obtained by removing the solvent in vacuo, adding water to the residue, and extracting the product with methylene chloride. This solution was dried (anhydrous sodium sulfate) and the solvent removed to give a brown oil which was treated with 10 ml of ether. The product which crystallized was filtered and washed sequentially with ether and hexane to give 78.0 g (91% of theory) of a beige powder, m.p. 121-122°C.

c) 5,11-Dihydro-11-[(4-methoxyphenyl)methyl]-6H-dipyrido[3,2-b:2',3'-e][1,4]diazepin-6-one

1.44 g of a 50% dispersion of sodium hydride in mineral oil was added to a solution of 3.69 g (0.010 mol) N-(2-chloro-3-pyridinyl)-2-[[(4-methoxyphenyl)methyl]amino]-3-pyridinecarboxamide in 100 ml of dimethylformamide. After the evolution of hydrogen stopped, the mixture was heated (110°C.) for 16 hours and then refluxed for eight hours. After cooling the mixture, the excess sodium hydride was decomposed by the slow addition of ice. The mixture was further diluted with water and the product extracted with ether and concentrated. The crystallized residue was filtered, washed with ether to give 1.60 g of 5,11-dihydro-11-[(4-methoxyphenyl)methyl]-6H-dipyrido[3,2-b:2',3'-e][1,4]diazepin-6-one (50% of theory) as an off-white powder, m.p. 209-210°C.

d) 5,11-Dihydro-11-[4-methoxyphenyl)methy]-5-methyl-6H-dipyrido[3,2-b:2',3'-e][1,4]diazepin-6-one

10.0 g (0.030 mol) of 5,11-dihydro-11-[(4-methoxyphenyl)methyl]-6H-dipyrido[3,2-b:2',3'-e][1,4]diazepin-6-one was added to a flask containing 2.16 g of a 50% dispersion of sodium hydride in mineral oil and 100 ml of dimethylformamide The resulting mixture was stirred at room temperature for 30 min. and then heated to 50°C. for 30 min.

After cooling, 8.51 g (0.060 mol) of methyl iodide in 10 ml of dimethylformamide was added dropwise and the mixture allowed to stir at room temperature overnight. Excess sodium hydride was decomposed by the careful addition of ice. Water was then added and the product was extracted with ether, dried (anhydrous sodium sulfate) and concentrated to give 10.3 g (99% of theory) of 5,11-dihydro-11-[(4-methoxyphenyl)methyl]-5-methyl-6H-dipyrido-[3,2-b:2',3'-e][1,4]diazepin-6-one as a light yellow oil suitable for use in the next reaction.

e) 5,11-Dihydro-5-methyl-6H-dipyrido[3,2-b:2',3'-e][1,4]diazepin-6-one

50 ml of trifluoroacetic acid was added to 10.3 g (0.030 mol) of 5,11-dihydro-11-[(4-methoxyphenyl)methyl]-5-methyl-6H-dipyrido[3,2-b:2',3'-e] [1,4]diazepin-6-one and the mixture stirred for one hour at room temperature. The acid was removed in vacuo and the residue stirred for one hour with 0.5% ammonia. The solid was filtered and dried to give 6.70 g (98% of theory) of pure 5,11-dihydro-5-methyl-6H-dipyrido [3,2-b:2',3'-e][1,4]diazepin-6-one, m.p. 230-232°C.

f) 5,11-Dihydro-11-ethyl-5-methyl-6H-dipyrido[3,2-b:2',3'-e][1,4]diazepin-6-one

2.00 g of a 50% dispersion of sodium hydride in mineral oil was added to a solution of 5.75 g (0.025 mol) of 5,11-dihydro-5-methyl-6H-dipyrido [3,2-b:2',3,'-e][1,4]diazepin-6-one in 100 ml of dimethylformamide. When the evolution of hydrogen ceased, the mixture was heated to 50°C. for 30 min., and then cooled to room temperature. Next 7.80 g of ethyl iodide (neat) was added dropwise over 15 min. , and the resulting mixture allowed to stir overnight at room temperature. The excess sodium hydride was decomposed by

the careful addition of ice followed by water. The product was extracted with ether, dried (anhydrous sodium sulfate) and evaporated to yield 4.5 g (70% of theory) of 5,11-dihydro-11-ethyl-5-methyl-6H-dipyrido[3,2-b:2',3'-e][1,4]diazepin-6-one, m.p. 130-132°C.

EXAMPLE A

Capsules or Tablets

A-1

| Ingredients | Quantity |
|---|---|
| Compound of Example 2 | 50 mg |
| Starch | 160 mg |
| Microcrys, Cellulose | 90 mg |
| Sodium Starch Gluctate | 10 mg |
| Magnesium Stearate | 2 mg |
| Fumed colloidal silica | 1 mg |

A-2

| Ingredients | Quantity |
|---|---|
| Example 2 | 50 mg |
| Dicalcium Phosphate | 160 mg |
| Microcrys. Cellulose | 90 mg |
| Stearic acid | 5 mg |
| Sodium Starch Glycolate | 10 mg |
| Fumed colloidal silica | 1 mg |

The compound of Example 2 is blended into a powder mixture with the premixed excipient materials as identified above with the exception of the lubricant. The lubricant is then blended in and the resulting blend compressed into tablets or filled into hard gelatin capsules.

EXAMPLE B

Parenteral Solutions

| Ingredients | Quantity |
|---|---|
| Compound of Example 2 | 500mg |
| Tartaric acid | 1.5g |
| Benzyl Alcohol | 0.1% by weight |
| Water for injection | q.s. to 100ml |

The excipient materials are mixed with the water and thereafter the compound of Example 2 is added. Mixing is continued until the solution is clear. The pH of this solution is adjusted to 3.0 and is then filtered into the appropriate vials or ampoules and sterilized by autoclaving.

EXAMPLE C

Nasal Solutions

| Ingredients | Quantity |
|---|---|
| Compound of Example 2 | 100mg |
| Citric acid | 1.92g |
| Benzalkonium chloride | 0.025% by weight |
| EDTA | 0.1 % by weight |
| Polyvinylalcohol | 10% by weight |
| Water | q.s. to 100ml |

The excipient materials are mixed with the water and thereafter the compound of Example 2 is added and mixing is continued until the solution is clear. The pH of this solution is adjusted to 4.0 and is then filtered into the appropriate vials or ampoules.

## Claims

1. A 5,11-dihydro-6H-dipyrido[3,2-b; 2',3'-e][1,4]diazepin-6-one of the formula I

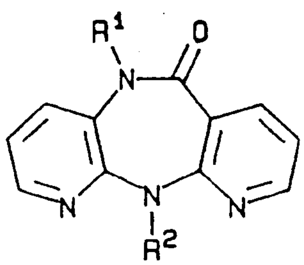

I

wherein, $R^1$ and $R^2$ are the same or different and are hydrogen or straight or branched alkyl of 1 to 5 carbon atoms, or a pharmaceutically acceptable acid addition salt thereof.

2. 5,11-dihydro-11-propyl-6H-dipyrido[3,2-b:2',3'-e][1,4]diazepin-6-one, or a pharmaceutically acceptable acid addition salt thereof according to claim 1.

3. 5,11-dihydro-5-methyl-11-propyl-6H-dipyrido[3,2-b:2',3'-e][1,4]diazepin-6-one, or a pharmaceutically acceptable acid addition salt thereof according to claim 1.

4. 5,11-diethyl-5,11-dihydro-6H-dipyrido[3,2-b:2',3'-e][1,4]diazepin-6-one, or a pharmaceutically acceptable acid addition salt thereof according to claim 1.

5. 5,11-Dihydro-11-ethyl-5-methyl-6H-dipyrido[3,2-b:2',3'-e][1,4]-diazepin-6-one, or a pharmaceutically acceptable salt thereof according to claim 1.

6. A pharmaceutical composition suitable for preventing or treating HIV-I infection which comprises a compound as set forth in claims 1, 2, 3, 4, or 5 and a pharmaceutically acceptable carrier.

7. The use of a compound according to any one of claims 1 to 5, in the manufacture of a pharmaceutical composition for preventing or treating HIV-1 infection in a human exposed to or infected by HIF-1.

8. A method of preparing a compound as defined in any one of claims 1 to 5, which comprises carrying out one of the following methods;

A. for preparing compounds in accordance with formula 1 in which $R^2$ is other than hydrogen, cyclizing a carboxylic acid amide of general formula II

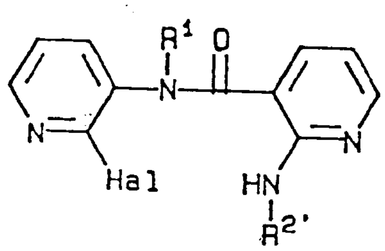

II

wherein $R^1$ has the same meanings as set forth with respect to Formula I, and $R^{2'}$ is straight or branched chain alkyl of 1 to 5 carbon atoms,

B. for producing compounds in accordance with Formula I in which $R^1$ has the same meanings as set forth with respect to Formula I and $R^2$ is hydrogen, hydrolytically cleaving the aryl methyl group in a compound of general Formula III

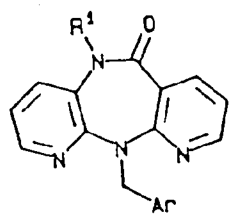

(III)

in which $R^1$ is as defined above and Ar is a phenyl or 4-methoxyphenyl group,

C. for preparing compounds in accordance with Formula I in which $R^1$ is other than hydrogen, by converting a 5,11-dihydro-6H-dipyrido [3,2-b:2',3'-e][1,4] diazepin-6-one of the Formula IV

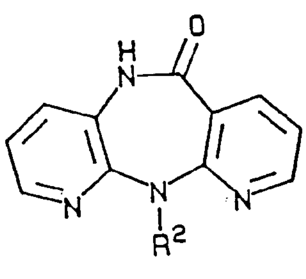

(IV)

wherein $R^2$ is as defined above, into the corresponding 5-alkali or alkaline earth metal compound and subsequently reacting the alkali metal compound with a reactive ester of the Formula V

$R^{1'}X$ (V)

wherein $R^{1'}$ is straight or branched chain alkyl of 1 to 5 carbon atoms and X is the radical of a reactive ester, a halogen atom, the group $OSO_2OR^{1'}$, the methanesulfonyloxy or ethane-sulfonyloxy group or an aromatic sulfonyloxy group,

C'. for preparing compounds in accordance with Formula I in which $R^1$ is other than hydrogen, by reacting a compound of Formula IV as defined above with a compound of Formula V as defined above in the presence of an amine or of an alkali carbonate or bicarbonate.

D. for preparing a compound of formula I in which $R^2$ is a straight or branched alkyl group of 1 to 5 carbon atoms, by converting a compound of Formula I in which $R^2$ is hydrogen into the corresponding metal salts of general Formula VIa or, when $R^1$ is hydrogen - of Formula VIb

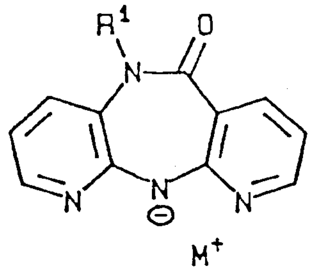

VIa

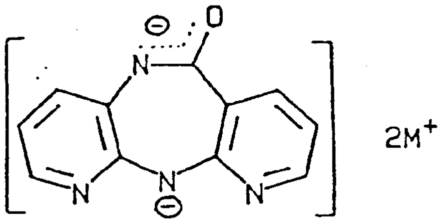

VIb

wherein $R^1$ is as hereinbefore defined and M represents an alkali metal, or M represents the group MgHal wherein Hal is a chlorine, bromine or iodine atom, and subsequently alkylating with a compound of the general Formula VII

$$R^{2''}X \qquad VII$$

wherein $R^{2''}$ is a straight or branched alkyl group of 1 to 5 carbon atoms and X is as defined above and thereafter isolating the compound of Formula I obtained by the method A, B, C, C' or D as such or as a pharaceutically acceptable acid addition salt thereof.

## Patentansprüche

1. 5,11-Dihydro-6H-dipyrido[3,2-b:2',3'-e][1,4]diazepin-6-on der Formel I

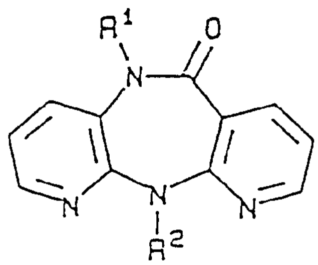

I,

worin $R^1$ und $R^2$ gleich oder verschieden sind und Wasserstoff oder gerades oder verzweigtes Alkyl mit 1 bis 5 Kohlenstoffatomen sind, oder ein pharmazeutisch annehmbares Säureadditionssalz davon.

2. 5,11-Dihydro-11-propyl-6H-dipyrido[3,2-b:2',3'-e][1,4]diazepin-6-on oder ein pharmazeutisch annehmbares Säureadditionssalz davon, gemäss Anspruch 1.

3. 5,11-Dihydro-5-methyl-11-propyl-6H-dipyrido[3,2-b:2',3'-e][1,4]diazepin-6-on oder ein pharmazeutisch annehmbares Säureadditionssalz davon, gemäss Anspruch 1.

4. 5,11-Diäthyl-5,11-dihydro-6H-dipyrido[3,2:b,2',3'-e] [1,4]diazepin-6-on oder ein pharmazeutisch annehmbares Säureadditionssalz davon, gemäss Anspruch 1.

5. 5,11-Dihydro-11-äthyl-5-methyl-6H-dipyrido[3,2-b:2',3'-e][1,4]diazepin-6-on oder ein pharmazeutisch annehmbares Säureadditionssalz davon, gemäss Anspruch 1.

6. Pharmazeutische Zusammensetzung, geeignet, um HIV-1 Infektionen vorzubeugen oder zu heilen, welche eine Verbindung wie in Ansprüchen 1,2,3,4, oder 5 dargelegt und einen pharmazeutisch annehmbaren Träger umfasst.

7. Verwendung einer Verbindung gemäss einem der Ansprüche 1 bis 5, bei der Herstellung einer pharmazeutischen Zusammensetzung zur Vorbeugung oder Behandlung von HIV-1 Infektionen in einem Menschen, der HIV-1 ausgesetzt wurde oder damit infiziert wurde.

8. Zubereitungsverfahren einer Verbindung wie sie in einem der Ansprüche 1 bis 5 definiert ist, welches umfasst, dass eines der folgenden Verfahren ausgeführt wird;

A. um Verbindungen in Übereinstimmung mit Formel I zuzubereiten, in welcher $R^2$ nicht Wasserstoff ist, durch Cyclisierung eines Carbonsäureamids der allgemeinen Formel II

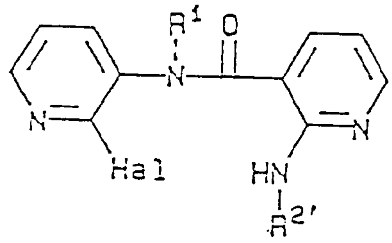

II,

worin $R^1$ die gleiche Bedeutung wie im Bezug auf Formel I dargelegt, besitzt und $R^{2'}$ ein geradkettiges oder verzweigtes Alkyl mit 1 bis 5 Kohlenstoffatomen ist,

B. um Verbindungen in Übereinstimmung mit Formel I herzustellen, in welcher $R^1$ die gleiche Bedeutung, wie im Bezug auf Formel I dargelegt, besitzt und $R^2$ Wasserstoff ist, durch hydrolytische Spaltung der Arylmethylgruppe in einer Verbindung der allgemeinen Formel III

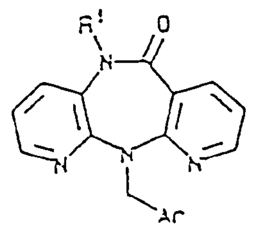

(III),

in welcher $R^1$ wie oben definiert ist und Ar eine Phenyl- oder 4-Methoxyphenylgruppe ist,

C. um Verbindungen in Übereinstimmung mit Formel I herzustellen, in welcher $R^1$ nicht Wasserstoff ist, durch Umwandlung eines 5,11-Dihydro-6H-dipyrido[3,2-b:2',3'-e][1,4]diazepin-6-ons der Formel IV

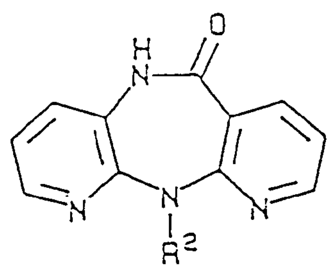

(IV),

worin $R^2$ wie oben definiert ist, in eine entsprechende 5-Alkali- oder Erdalkalimetallverbindung und nachfolgender Umsetzung der Alkalimetallverbindung mit einem reaktiven Ester der Formel V

$$R^{1'}X \quad (V),$$

worin $R^1$ ein geradkettiges oder verzweigtes Alkyl mit 1 bis 5 Kohlenstoffatomen ist und X ein Radikal eines reaktiven Esters, ein Halogenatom, die Gruppe $OSO_2OR^{1'}$, die Methansulfonyloxy- oder Äthansufonyloxygruppe oder eine aromatische Sulfonyloxygruppe ist,

C'. um Verbindungen in Übereinstimmung mit Formel I zuzubereiten, in welcher $R^1$ nicht Wasserstoff ist, durch Reaktion einer Verbindung der Formel IV, wie oben definiert, mit einer Verbindung der Formel V, wie oben definiert, im Beisein eines Amins oder eines Alkalicarbonats oder -bicarbonats.

D. um eine Verbindung der Formel I zuzubereiten, in welcher $R^2$ eine gerade oder verzweigte Alkylgruppe mit 1 bis 5 Kohlenstoffatomen ist, durch Umwandlung einer Verbindung der Formel I, in welcher $R^2$ Wasserstoff ist in die entsprechenden Metallsalze der allgemeinen Formel VIa oder wenn $R^1$ Wasserstoff ist - der Formel VIb

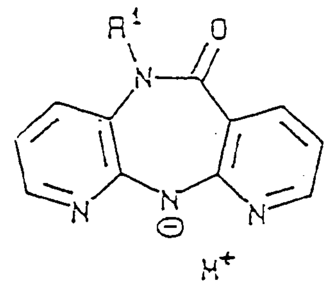

VIa

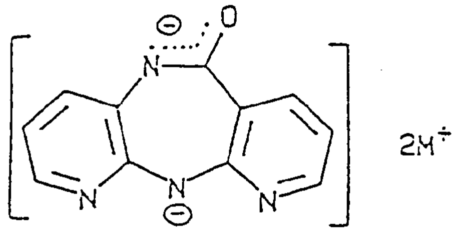

VIb

worin $R^1$ wie hierin zuvor definiert ist und M ein Alkalimetall darstellt, oder M die Gruppe MgHal darstellt, worin Hal ein Chlor-, Brom- oder Jodatom ist und nachfolgender Alkylierung mit einer Verbindung der allgemeinen Formel VII

$$R^{2''}X \qquad (VII),$$

worin $R^{2''}$ eine gerade oder verzweigte Alkylgruppe mit 1 bis 5 Kohlenstoffatomen ist und X wie oben definiert ist

und danach Isolierung der Verbindungen der Formel I, die durch die Verfahren A, B, C, C' oder D als solches, oder als ein pharmazeutisch annehmbares Säureadditionssalz davon, erhalten wurden.

## Revendications

1. 5,11-dihydro-6H-dipyrido[3,2-b: 2',3'-e][1,4]diazépine-6-one de formule I

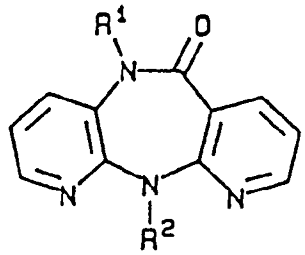

I

dans laquelle $R^1$ et $R^2$ sont identiques ou différents et sont l'hydrogène ou un groupe alkyle linéaire ou ramifié de 1 à 5 atomes de carbone, ou l'un de ses sels d'addition d'acide acceptables du point de vue pharmaceutique.

2. 5,11-dihydro-11-propyl-6H-dipyrido[3,2-b: 2',3'-e][1,4]diazépine-6-one ou l'un de ses sels d'addition d'acide acceptables du point de vue pharmaceutique selon la revendication 1.

3. 5,11-dihydro-5-méthyl-11-propyl-6H-dipyrido[3,2-b: 2',3'-e][1,4]-diazépine-6-one ou l'un de ses sels d'addition d'acide acceptables du point de vue pharmaceutique selon la revendication 1.

4. 5,11-diéthyl-5,11-dihydro-6H-dipyrido[3,2-b: 2',3'-e][1,4]-diazépine-6-one ou l'un de ses sels d'addition d'acide acceptables du point de vue pharmaceutique selon la revendication 1.

5. 5,11-dihydro-11-éthyl-5-méthyl-6H-dipyrido[3,2-b: 2',3'-e][1,4]-diazépine-6-one ou l'un de ses sels d'addition d'acide acceptables du point de vue pharmaceutique selon la revendication 1.

6. Composition pharmaceutique appropriée pour la prévention ou le traitement des infections par HIV-I qui comprend un composé selon les revendications 1, 2, 3, 4 ou 5 et un véhicule acceptable du point de vue pharmaceutique.

7. Utilisation d'un composé selon l'une quelconque des revendications 1 à 5 pour la préparation d'une composition pharmaceutique pour la prévention ou le traitement des infections par HIV-I chez un humain exposé à ou infecté par HIV-I.

8. Procédé de préparation d'un composé tel que défini dans l'une quelconque des revendications 1 à 5, qui comprend la mise en oeuvre de l'un des procédés suivants:

A. pour préparer des composés selon la formule 1 dans laquelle $R^2$ est différent de l'hydrogène, la cyclisation d'un amide d'acide carboxylique de formule générale II

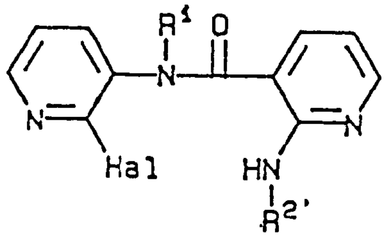

II

dans laquelle $R^1$ a les mêmes significations que celles données à propos de la formule I, et $R^{2'}$ est un groupe alkyle à chaîne linéaire ou ramifiée de 1 à 5 atomes de carbone,

B. pour produire des composés selon la formule I dans laquelle $R^1$ a les mêmes significations que celles données à propos de la formule I et $R^2$ est l'hydrogène, le clivage par hydrolyse du groupe arylméthyle d'un composé de formule générale III

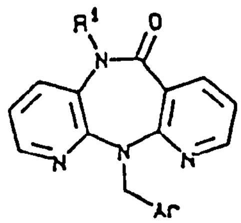

(III)

dans laquelle $R^1$ est tel que défini ci-dessus et Ar est un groupe phényle ou 4-méthoxyphényle,

C. pour préparer des composés selon la formule I dans laquelle $R^1$ est différent de l'hydrogène, par conversion d'une 5,11-dihydro-6H-dipyrido[3,2-b:2',3'-e][1,4]diazépine-6-one de formule IV

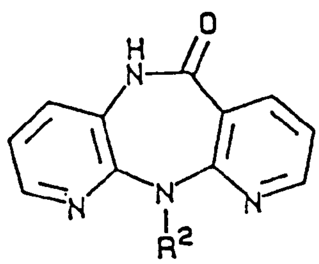

(IV)

dans laquelle $R^2$ est tel que défini ci-dessus, en le composé 5-alcalin ou 5-alcalinoterreux correspondant puis réaction du composé alcalin avec un ester réactif de formule V

$R^{1'}X$ (V)

dans laquelle $R^{1'}$ est un groupe alkyle à chaîne linéaire ou ramifiée de 1 à 5 atomes de carbone et X est le radical d'un ester réactif, un atome d'halogène, le groupe $OSO_2OR^{1'}$, le groupe méthanesulfonyloxy ou éthanesulfonyloxy ou un groupe sulfonyloxy aromatique,

C'. pour préparer des composés selon la formule I dans laquelle $R^1$ est différent de l'hydrogène, par réaction d'un composé de formule IV tel que défini ci-dessus avec un composé de formule V tel que défini ci-dessus en présence d'une amine ou d'un carbonate ou bicarbonate alcalin,

D. pour préparer un composé de formule I dans laquelle $R^2$ est un groupe alkyle à chaîne linéaire ou ramifiée de 1 à 5 atomes de carbone, par conversion d'un composé de formule I dans laquelle $R^2$ est l'hydrogène en les sels métalliques correspondants de formule générale VIa ou, lorsque $R^1$ est l'hydrogène, de formule VIb

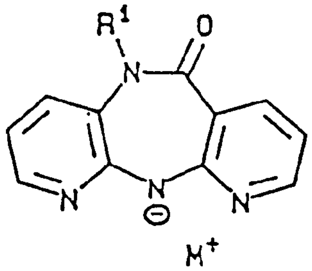

VIa

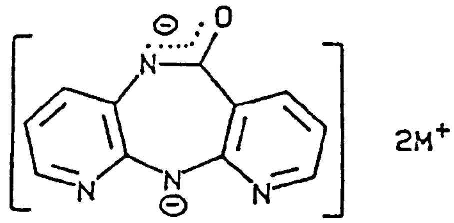

VIb

dans laquelle $R^1$ est tel que défini précédemment et M représente un métal alcalin, ou bien M représente le groupe MgHal dans lequel Hal est un atome de chlore, de brome ou d'iode, puis alkylation avec un composé de formule générale VII

$$R^{2''}X \qquad (VII)$$

dans laquelle $R^{2''}$ est un groupe alkyle linéaire ou ramifié de 1 à 5 atomes de carbone et X est tel que défini ci-dessus puis isolement du composé de formule I obtenu par le procédé A, B, C, C' ou D en l'état ou sous forme de l'un de ses sels d'addition d'acide acceptables du point de vue pharmaceutique.